(19) Europäisches Patentamt
European Patent Office
Office européen des brevets

(11) Publication number: 0 361 840 B1

(12) **EUROPEAN PATENT SPECIFICATION**

(45) Date of publication of patent specification: **26.04.95**

(51) Int. Cl.$^6$: **C07C 63/38**, C07C 51/265

(21) Application number: **89309750.1**

(22) Date of filing: **26.09.89**

(54) **Method for making 2,6-naphthalene-dicarboxylic acid.**

(30) Priority: **28.09.88 US 250837**

(43) Date of publication of application:
**04.04.90 Bulletin 90/14**

(45) Publication of the grant of the patent:
**26.04.95 Bulletin 95/17**

(84) Designated Contracting States:
**BE DE FR GB IT NL**

(56) References cited:
**EP-A- 0 257 788**
**EP-A- 0 323 309**
**GB-A- 910 484**
**GB-A- 2 187 744**

(73) Proprietor: **AMOCO CORPORATION**
**200 East Randolph Drive**
**Chicago**
**Illinois 60601 (US)**

(72) Inventor: **Young, David Allen**
**3 South 532 Tinker**
**Warrenville**
**Illinois 60555 (US)**

(74) Representative: **Ritter, Stephen David et al**
**Mathys & Squire**
**100 Grays Inn Road**
**London WC1X 8AL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 361 840 B1

## Description

This invention relates to a method for making 2,6-naphthalenedicarboxylic acid by the oxidation of 2,6-diethylnaphthalene or its oxidation derivative with an oxygen-containing gas under liquid-phase conditions and in the presence of a catalyst comprising metal and bromine components.

### Discussion of the Prior Art

Fibers and films produced from polyethylenenaphthalate have improved strength and thermal properties relative to fibers and films produced from polyethyleneterephthalate and are especially useful in applications such as tire cords, magnetic tape backings and hot-fill containers. However, the use of 2,6-naphthalenedicarboxylic acid is inhibited by its relatively high cost, which is due to the relative unavailability and high cost of the preferred feedstock, 2,6-dimethylnaphthalene, which can be readily oxidized to 2,6-naphthalenedicarboxylic acid under conditions that are conventional for the oxidation of alkylated aromatics -- that is, under liquid phase conditions, in a solvent, at elevated temperature and pressure, by an oxygen-containing gas, and in the presence of a catalyst comprising cobalt, manganese and bromine components.

It has been proposed to substitute 2,6-diethylnaphthalene or its oxidation derivative for 2,6-dimethylnaphthalene or its oxidation derivative in the aforesaid conventional oxidation. For example, Japanese examined, accepted patent application 73027318 discloses a method for the production of a naphthalenedicarboxylic by the oxidation of a dialkylnaphthalene having an ethyl or isopropyl group as at least one of the alkyl substituents with oxygen in an acetic acid medium at a reaction temperature of 90-160°C and partial oxygen pressure of less than 0.5 atmosphere absolute, and in the presence of a catalyst having cobalt, manganese and bromine components. At least 2.5 parts of the acetic acid solvent are employed per part of dialkylnaphthalene and the cobalt, manganese and bromine components of the catalyst are employed in relative amounts that satisfy the three equations:
(a) $x + y > 2.0$, (b) $y \geqq 0.15/x$, and (c) $0.2 \leqq z/(x+y) \leqq 10$, where x, y and z are the weight percent of cobalt, manganese and bromine, respectively, with respect to the weight of dialkylnaphthalene.

In addition, Hirose et al. (Teijin Petrochemical Industries Ltd.), published European Patent Application No. 142719, disclose that the use of the aforesaid conventional oxidation conditions for the oxidation of 2,6-diisopropylnaphthalene, or its oxidation derivative, results in an extremely low yield of 2,6-naphthalenedicarboxylic acid, which is also of low purity because of the formation of relatively large amounts of undesirable by-products. This published European Patent Application also discloses that variation of the aforesaid conventional oxidation, either by the use therein of a plurality of oxidation stages in which the reaction temperature is increased stepwise or continuously from a relatively low temperature in an early stage to relatively higher temperatures in a latter stage, or by maintaining a low concentration of 2,6-diisopropylnaphthalene or its oxidation derivative in the solvent in the oxidation, did not afford acceptable yields of 2,6-naphthalendicarboxylic acid.

By contrast, the method of the invention disclosed and claimed in Hayaski et al., United Kingdom Patent Application No. 2187744A is a process for the preparation of 2,6-naphthalenedicarboxylic acid by the oxidation of 2,6-diisopropylnaphthalene or an oxidized intermediate thereof with molecular oxygen in a lower aliphatic monocarboxylic acid solvent containing no more than 30 weight percent of water, at a reaction temperature of 140-210°C and in the presence of a catalyst comprising a water-soluble cerium salt, a water-soluble cobalt and/or manganese salt and a bromine-containing compound. The molar ratio of diisopropylnaphthalene or its oxidized intermediate-to-the heavy metals of the catalyst is less than 0.4. The atomic ratio of bromine-to-the total heavy metals is 0.001-1, and the usual atomic ratio of the water-soluble salt of cobalt, manganese or their mixture-to-the water soluble salt of cerium is 0.03-30.

Hayaski et al., U.S. Patent No. 4,754,060 discloses a method for producing 2,6-naphthalenedicarboxylic acid together with trimellitic acid by oxidation of 2,6-diisopropylnaphthalene in a lower aliphatic monocarboxylic acid solvent containing not more than 30 weight percent of water with molecular oxygen at 140-210°C and in the presence of a catalyst comprising cobalt, manganese, or a mixture thereof, and bromine. The amount of bromine employed is 0.0001-0.01 in an atomic ratio to the heavy metal catalyst components. The amount of cobalt, manganese or a mixture thereof employed is preferably 0.005-0.2 mole to 100 grams of the solvent.

Furthermore, each of Hirose et al., U.S. Patent No. 4,709,088 and European Patent Application No. 142719 discloses a method for producing 2,6-naphthalenedicarboxylic acid by the oxidation of 2,6-diisopropylnaphthalene in the presence of a relatively large amount of an oxidation catalyst containing a heavy metal element selected from the group consisting of cobalt and manganese and a bromine component, with 0.2 to 10 gram-atoms of the heavy metal element being used per mole of 2,6-diisopropylnaphthalene or its oxidation derivative. The oxidation is performed at 140-210 °C, with the solvent employed at a level of at least twice the total weight of 2,6-diisopropylnaphthalene, and preferably with an atomic ratio of cobalt-to-manganese of 10:90 to 95:5 and with an atomic ratio of bromine to heavy metal components of 0.05:1 to 0.5:1.

Furthermore, published Japanese patent application Kokai No. 120342/87 discloses a process for oxidizing 2,6-diisopropylnaphthalene or its oxidation derivative to 2,6-naphthalenedicarboxylic acid in a reaction medium containing at least 50 weight percent of propionic acid and in the presence of a catalyst comprising (1) a bromine element, (2) cobalt or manganese or a mixture thereof, and (3) an alkali metal element.

Published Japanese patent application Kokai No. 120343/87 discloses a process that is very similar to that of published Japanese patent application Kokai No. 120342/87 but in which the solvent contains at least 50 weight percent of at least one monocarboxylic acid selected from butyric acid, valeric acid and benzoic acid.

Each of Hirose, U.S. Patent No. 4,716,245 and published Japanese patent application Kokai No. 246143/86 discloses a process that is very similar to that of published Japanese patent application Kokai No. 120342/87 except that at least 70 weight percent of the solvent is acetic acid or propionic acid or a mixture thereof and except that 1.1 to 15 gram atoms of the alkali metal component of the catalyst must be employed per gram atom of the bromine component of the catalyst.

## OBJECTS OF THE INVENTION

It is therefore a general object of the present invention to provide an improved method for oxidizing 2,6-diethylnaphthalene or its oxidation derivative to 2,6-naphthalenedicarboxylic acid which overcomes the aforementioned problems of the prior art.

More particularly, it is an object of the present invention to provide an improved method for oxidizing 2,6-diethylnaphthalene or its oxidation derivative to 2,6-naphthalenedicarboxylic acid with an improved yield of 2,6-naphthalenedicarboxylic

It is another object of the method of the present invention to provide an improved method for oxidizing 2,6-diethylnaphthalene or its oxidation derivative to 2,6-naphthalenedicarboxylic acid of improved purity.

Other objects and advantages of the present invention will become apparent upon reading the following detailed description and appended claims.

## SUMMARY OF THE INVENTION

These objects are achieved by the improved method of this invention for producing 2,6-naphthalenedicarboxylic acid comprising: exothermically oxidizing 2,6-diethylnaphthalene or its oxidation derivative as the starting material with an oxygen-containing gas in the liquid phase in a solvent comprising an aliphatic monocarboxylic acid having, in an oxidation reactor at a temperature of 165 °C-270 °C and a pressure of 101-3534 kPa (0-35 kg/cm$^2$ gauge) and in the presence of an oxidation catalyst comprising cobalt, manganese, bromine and cerium components, wherein the atom ratio of cobalt (calculated as elemental cobalt) in the cobalt component of the catalyst-to-the starting material is in the range of from 30 to 10000 mga per gram mole of the starting material, wherein the atom ratio of manganese (calculated as elemental manganese) in the manganese component of the catalyst-to-cobalt (calculated as elemental cobalt) in the cobalt component of the catalyst is in the range of from 0.5 to 3 mga per mga of cobalt, wherein the atom ratio of bromine (calculated as elemental bromine) in the bromine component of the catalyst-to-total cobalt and manganese (calculated as elemental cobalt and elemental manganese) in the cobalt and manganese components of the catalyst is in the range of from 0.05 to 1 mga per mga of total cobalt and manganese, wherein the atom ratio of cerium (calculated as elemental cerium) in the cerium component of the catalyst-to-cobalt (calculated as elemental cobalt) in the cobalt component of the catalyst is in the range of from 0.025 to 1.0 mga per mga of cobalt, and wherein heat generated in the oxidation reactor is at least partially dissipated by vaporization of liquids therein and withdrawal of the resulting vapors from the oxidation reactor.

## DETAILED DESCRIPTION OF THE PREFERRED EMBODIMENTS

The starting material employed in the method of this invention is represented by the following general formula:

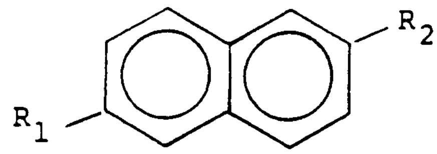

wherein $R_1$ is selected from the group consisting of

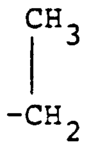 , 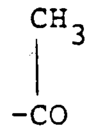 , 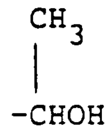 , 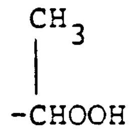

and wherein $R_2$ is selected from the group consisting of

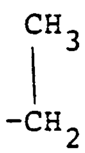 , 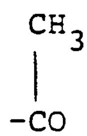 , 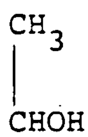 , 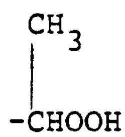 ,

-COOH and -CHO
and wherein $R_1$ and $R_2$ can be the same or different from each other. Preferably the starting material is 2,6-diethylnaphthalene.

Suitable solvents for use in the method of the present invention for producing 2,6-naphthalenedicarboxylic acid include any aliphatic $C_2$-$C_6$ monocarboxylic acid such as acetic acid, propionic acid, n-butyric acid, isobutyric acid, n-valeric acid, trimethylacetic acid, and caproic acid and mixtures thereof with water. Preferably, the solvent is a mixture of acetic acid and water, which more preferably contains from 1 to 30 weight percent of water and most preferably from 2 to 15 weight percent of water, the total amount of both that is introduced into the oxidation reactor from an external source and that is generated in situ in the oxidation reaction. Heat generated in the highly exothermic liquid-phase oxidation is dissipated at least partially by vaporization of solvent in the oxidation reactor and withdrawal of at least some of the solvent from the reactor as a vapor, which is then condensed and recycled to the reactor. In addition, some solvent is withdrawn from the reactor as a liquid in the product stream. After separation of the 2,6-naphthalene dicarboxylic acid product from the product stream, at least a portion of the mother liquor (solvent) in the resulting product stream is recycled to the reactor. The weight ratio of the total amount of the monocarboxylic acid solvent (both fresh and recycle)-to-total amount of 2,6-diethylnaphthalene or its oxidation derivative added during the entire oxidation step in the method of this invention is from about 2:1, preferably from about 3:1, up to about 20:1, preferably up to about 10:1.

The source of molecular oxygen employed in the method of this invention can vary in molecular oxygen content from that of air to oxygen gas. Air is the preferred source of molecular oxygen. Typically, as in liquid-phase oxidations of alkylaromatics such as p-xylene by oxygen and in the presence of a catalyst having heavy metal and bromine components, the oxygen-containing gas fed to the oxidation reactor in the method of the present invention provides a pressure of oxygen in the oxidation reactor of from 50 to 300 pounds per square inch gauge and an exhaust gas-vapor mixture containing from 0.5 to 8 volume percent oxygen and preferably from 2 to 6 volume percent oxygen (measured on a solvent-free basis).

The catalyst employed in the method of this invention for producing 2,6-naphthalenedicarboxylic acid comprises cobalt, manganese, bromine, and cerium components. The atom ratio of cobalt (calculated as elemental cobalt) in the cobalt component of the catalyst-to-the starting material in the liquid-phase oxidation is in the range of from about 30, preferably from about 60, to about 10000, preferably to about 500, milligram atoms (mga) per gram mole of the starting material. The atom ratio of manganese (calculated as elemental manganese) in the manganese component of the catalyst-to-cobalt (calculated as elemental cobalt) in the cobalt component of the catalyst in the liquid-phase oxidation is in the range of from 0.5, preferably from 1.0, to 3, preferably to 2.5, mga per mga of cobalt. The atom ratio of bromine (calculated as elemental bromine) in the bromine component of the catalyst-to-total cobalt and manganese (calculated as elemental cobalt and elemental manganese) in the cobalt and manganese components of the catalyst is in

the range of from 0.05, preferably from 0.075, to 1, preferably to 0.4, mga per mga of total cobalt and manganese. The atom ratio of cerium (calculated as elemental cerium) in the cerium component of the catalyst-to-cobalt (calculated as elemental cobalt) in the cobalt component of the catalyst is in the range of from 0.025, preferably from 0.05, to 1.0, preferably to 0.6, mga per mga of cobalt.

Each of the cobalt, manganese, bromine, and cerium components of the catalyst employed in the method of this invention can be provided in any of its known ionic or combined forms that provide soluble forms of cobalt, manganese, bromine, and cerium, respectively, in the solvent in the oxidation reactor. For example, when the solvent is an acetic acid medium, cobalt and/or manganese and/or cerium carbonate, acetate, and/or bromide can be employed. The 0.05:1 to 1:1 bromine-to-total cobalt and manganese milligram atom ratio is provided by a suitable source of bromine. Such bromine sources include elemental bromine ($Br_2$), or ionic bromides (for example, HBr, NaBr, KBr, $NH_4Br$, etc.), or organic bromides which are known to provide bromide ions at the operating temperature of the oxidation (e.g., bromobenzenes, benzylbromide, mono- and dibromoacetic acid, bromoacetyl bromide, tetrabromoethane, ethylene-di-bromide, etc.). The total bromine in molecular bromine and ionic bromide is used to determine satisfaction of the elemental bromine-to-total cobalt and manganese milligram atom ratio of 0.05:1 to 1:1. The bromide ion released from the organic bromides at the oxidation operating conditions can be readily determined by known analytical means. Tetrabromoethane, for example, at operating temperatures of 170° to 225°C has been found to yield about 3 effective gram atoms of bromine per gram mole.

In operation, the minimum pressure at which the oxidation reactor is maintained is that pressure which will maintain a substantial liquid phase of starting material and at least 70 percent of the solvent. When the solvent is an acetic acid-water mixture, reaction gauge pressures in the oxidation reactor are in the range 101 - 3534 kPa (0 kg/cm$^2$ to 35 kg/cm$^2$), and typically are in the range 1082 - 3043 kPa (10 kg/cm$^2$ to 30 kg/cm$^2$). The temperature range within the oxidation reactor is from 165°C, preferably from 170°C, to 270°C, 270°C, preferably to 200°C. The solvent residence time in the oxidation reactor is generally from about 20 to about 300 minutes and preferably from about 45 to about 120 minutes.

The oxidation of the method of this invention can be performed either on a batch, continuous, or semi-continuous mode. In the batch mode, the starting material, solvent and the catalyst components are initially introduced batchwise into the reactor, and the temperature and pressure of the reactor contents are then raised to the desired levels therefor for the commencement of the oxidation reaction. Air is introduced continuously into the reactor. After commencement of the oxidation reaction-for example, after all of the starting material had been completely introduced into the reactor, the temperature of the reactor contents is raised. In the continuous mode, each of the starting material, air, solvent and the catalyst components dissolved in the solvent are continuously introduced through a first inlet or set of inlets into a first oxidation reactor where the temperature and pressure are at the desired levels therefor for initiation of the oxidation reaction; and a product stream comprising the 2,6-naphthalene dicarboxylic acid product and catalyst components dissolved in the solvent is withdrawn from the reactor. In the semi-continuous mode, the solvent and the catalyst components are initially introduced batchwise into the reactor, and then the starting material in solvent and air are introduced continuously into the reactor. After commencement of the oxidation reaction, the temperature of the reactor contents is raised. Typically, the semi-continuous mode is employed for the oxidation of the method of this invention, with the temperature of the reactor contents preferably at about 165°-205°C when starting material is first introduced and rising to a steady-state temperature of preferably about 170°C-270°C as the exothermic oxidation proceeds and with the starting material being introduced preferably at 0.05-1.0 parts per part of solvent by weight per hour preferably for 0.3-4.0 hours.

In each case, the progress of the reaction is monitored by measuring oxygen uptake and temperature changes. A run is terminated after oxygen uptake ceases, as evidenced by a rapid decrease in oxygen uptake--that is, by a rapid increase in the oxygen concentration in the vapor-gas mixture withdrawn from the reactor.

Thereafter, the product stream in the continuous mode or the reactor contents in the batch or semi-continuous mode are cooled at a rate of about 25°C-140°C per hour to a temperature in the range of from about 35°C to about 120°C in at least one step and in at least one crystallizer such that essentially all of the resulting crude, solid 2,6-naphthalenedicarboxylic acid product is separated from the product mixture typically by filtration or centrifugation at a temperature in the range of from about 35°C to about 120°C. The use of lower temperatures results in the recovery of a significantly less pure product and the use of higher temperatures results in the recovery of less product.

The present invention will be more clearly understood from the following specific example, which involves the oxidation of 2,6-diethylnaphthalene on a semi-continuous basis. The reactor employed was a 1-liter reactor equipped with a stirrer, air line, and a line for introduction of air during the oxidation. The

temperature of the reactor was controlled by insulated electrical heaters which surrounded the autoclave. The vented gases from the reactor were passed through a condenser, cooled by dry-ice, and then through instruments which recorded the gaseous flow rate and the concentration of oxygen and carbon oxides in the gas stream. 250 grams of acetic acid solvent and 4.7 grams of cobalt (II) acetate tetrahydrate, 9.3 grams of manganese (II) acetate tetrahydrate, 0.67 gram of cerium (III) acetate, 1.5 hydrate, and 0.67 gram of sodium bromide components of the catalyst were introduced batchwise into the reactor. The reactor was purged and then pressurized to 2758 kPa (400 pounds per square inch gauge) with a slow addition of nitrogen gas. The temperature of the reactor contents was raised to 193-197 °C, the desired level therefor for commencement of the oxidation, and then 170 milliliters of a solution of 0.48 gram of 2,6-diethylnaphthalene per milliliter of acetic acid at a rate of 2-2.5 milliliters per minute and a solution of 20.9 volume percent oxygen in nitrogen at a rate of $1 \cdot 4 \times 10^{-4}$ $m^3/s$ (18 cubic feet per hour) were introduced continuously into the reactor. Immediately after the introduction of the 2,6-diethylnaphthalene was completed, (which required 75 minutes). and the introduction of the oxygen-nitrogen solution was continued for about 7.5 minutes longer. The pressure of the reactor was controlled by a research control valve. The rate of oxidation was determined by measuring the oxygen content of the vent gas and knowing the flow rate of air through the reactor, and was employed as a measure of the extent of conversion of the reactant. The reaction was terminated after oxygen uptake had ceased and the oxygen content of the vent gas reached a steady value of 12.0 volume percent, whereupon the flow of air into the reactor was replaced by a flow of nitrogen gas into the reactor. The yield of 2,6- naphthalenedicarboxylic was measured as 90 mole % based on the amounts of 2,6- naphthalenedicarboxylic acid in the reaction product slurry and reactor wash. The 5 mole percent yield of trimellitic acid represents the moles of trimellitic acid identified in the reaction product slurry per mole of 2,6-diethylnaphthalene used.

## Claims

1. A method for producing 2,6-naphthalene dicarboxylic acid comprising: exothermically oxidizing 2,6-diethylnaphthalene or its oxidation derivative as the starting material with an oxygen-containing gas in the liquid-phase in a solvent comprising an aliphatic monocarboxylic acid, in an oxidation reactor at a temperature of 165 °C-270 °C and a pressure of 101-3534 kPa (0-35 $kg/cm^2$ gauge) and in the presence of an oxidation catalyst comprising cobalt, manganese, bromine and cerium components, wherein the atom ratio of cobalt (calculated as elemental cobalt) in the cobalt component of the catalyst-to-the starting material in the liquid-phase oxidation is in the range of from 30 to 10000 mga per gram mole of the starting material, wherein the atom ratio of manganese (calculated as elemental manganese) in the manganese component of the catalyst-to-cobalt (calculated as elemental cobalt) in the cobalt component of the catalyst is in the range of from 0.5 to 3 mga per mga of cobalt, wherein the atom ratio of bromine (calculated as elemental bromine) in the bromine component of the catalyst-to-total cobalt and manganese (calculated as elemental cobalt and elemental manganese) in the cobalt and manganese components of the catalyst is in the range of from 0.05 to 1 mga per mga of total cobalt and manganese, and wherein the atom ratio of cerium (calculated as elemental cerium) in the cerium component of the catalyst-to-cobalt (calculated as elemental cobalt) in the cobalt component of the catalyst is in the range of from 0.025 to 1.0 mga per mga of cobalt, wherein heat generated in the oxidation reactor is at least partially dissipated by vaporization of liquids therein and withdrawal of the resulting vapors from the oxidation reactor, and wherein oxygen is maintained at a concentration level in the oxidation reactor such that the concentration of oxygen in the aforesaid withdrawn vapors is in the range of from 0.1 to 15 volume percent.

2. The method of Claim 1 wherein the atom ratio of cobalt (calculated as elemental cobalt) in the cobalt component of the catalyst-to-the starting material in the liquid-phase oxidation is in the range of from 60 to 500 mga per gram mole of the starting material.

3. The method of Claim 1 or Claim 2 wherein the atom ratio of manganese (calculated as elemental manganese) in the manganese component of the catalyst-to-cobalt (calculated as elemental cobalt) in the cobalt component of the catalyst is in the range of from 1.0 to 2.5 mga per mga of cobalt.

4. The method of any preceding Claim wherein the atom ratio of bromine (calculated as elemental bromine) in the bromine (calculated as elemental bromine) in the bromine component of the catalyst-to-total cobalt and manganese (calculated as elemental cobalt and elemental manganese) in the cobalt and manganese components of the catalyst is in the range of from 0.075 to 0.4 mga per mga of total

cobalt and manganese.

5. The method of any preceding Claim wherein the atom ratio of cerium (calculated as elemental cerium) in the cerium component of the catalyst-to-cobalt (calculated as elemental cobalt) in the cobalt component of the catalyst is in the range of from 0.05 to 0.6 mga per mga of cobalt.

6. The method of any preceding Claim wherein the oxygen is maintained at a concentration level in the reactor such that the concentration of oxygen in the withdrawn vapors is in the range of from 0.05 to 8 volume percent.

7. The method of any preceding Claim wherein the oxidation is performed at a temperature in the range of from 165 ° C to 270 ° C.

8. The method of Claim 7 wherein the oxidation is performed at a temperature in the range of from 170 ° C to 200 ° C

9. The method of any preceding Claim wherein the solvent is a mixture of acetic acid and from 1 to 30 weight percent of water, based on the weight of acetic acid.

10. The method of Claim 9 wherein the solvent is a mixture of acetic acid and from 2 to 15 weight percent of water, based on the weight of acetic acid.

11. The method of any preceding Claim wherein the pressure is in the range of from 0 to 35 $kg/cm^2$ gauge.

12. The method of Claim 11 wherein the pressure is in the range of from 10 to 30 $kg/cm^2$ gauge.

**Patentansprüche**

1. Verfahren zur Herstellung von 2,6-Naphthalindicarbonsäure, welches umfaßt: exothermes Oxidieren von 2,6-Diethylnaphthalin oder dessen Oxidationsderivaten als Ausgangsmaterial mit einem Sauerstoff-enthaltenden Gas in flüssiger Phase in einem Lösungsmittel, das eine aliphatische Monocarbonsäure umfaßt, in einem Oxidationsreaktor bei einer Temperatur von 165 ° C - 270 ° C und einem Druck von 101 - 3534 kPa (0 - 35 $kg/cm^2$ Manometerdruck) und in Anwesenheit eines Oxidationskatalysators, der Kobalt-, Mangan-, Brom- und Cer-Komponenten enthält, wobei das Atomverhältnis von Kobalt (errechnet als elementares Kobalt) in der Kobalt-Komponente des Katalysators zu dem Ausgangsmaterial der Flüssigphasenoxidation im Bereich von 30 bis 1000 Milligramm-Atom pro Gramm-Mol des Ausgangsmaterials liegt, wobei das Atomverhältnis von Mangan (errechnet als elementares Mangan) in der Mangan-Komponente des Katalysators zu Kobalt (errechnet als elementares Kobalt) in der Kobalt-Komponente des Katalysators im Bereich von 0,5 bis 3 Milligramm-Atom pro Milligramm-Atom Kobalt liegt, wobei das Atomverhältnis von Brom (errechnet als elementares Brom) in der Brom-Komponente des Katalysators zu der Gesamtmenge an Kobalt und Mangan (errechnet als elementares Kobalt und elementares Mangan) in den Kobalt- und Mangan-Komponenten des Katalysators im Bereich von 0,05 bis 1 Milligramm-Atom pro Milligramm-Atom der Gesamtmenge an Kobalt und Mangan liegt, und wobei das Atomverhältnis von Cer (errechnet als elementares Cer) in der Cer-Komponente des Katalysators zu Kobalt (errechnet als elementares Kobalt) in der Kobalt-Komponente des Katalysators im Bereich von 0,025 bis 1,0 Milligramm-Atom pro Milligramm-Atom Kobalt liegt, wobei die in dem Oxidationsreaktor erzeugte Wärme teilweise durch Verdampfen der darin enthaltenen Flüssigkeiten und Abziehen der erhaltenen Dämpfe aus dem Reaktionsreaktor abgeleitet wird, und wobei der Sauerstoff in dem Oxidationsreaktor in einer solchen Konzentration gehalten wird, daß der Sauerstoffgehalt in den vorgenannten abgeleiteten Dämpfen im Bereich von 0,1 bis 15 Volumen-% liegt.

2. Verfahren nach Anspruch 1, worin das Atomverhältnis von Kobalt (errechnet als elementares Kobalt) in der Kobalt-Komponente des Katalysators zu dem Ausgangsmaterial in der Flüssigphasenoxidation im Bereich von 60 bis 500 Milligramm-Atom pro Gramm-Mol des Ausgangsmaterials liegt.

3. Verfahren nach Anspruch 1 oder 2, worin das Atomverhältnis von Mangan (errechnet als elementares Mangan) in der Mangan-Komponente des Katalysators zu Kolbat (errechnet als elementares Kobalt) in der Kobalt-Komponente des Katalysators im Bereich von 1,0 bis 2,5 Milligramm-Atom pro Milligramm-Atom Kobalt liegt.

**4.** Verfahren nach einem der vorhergehenden Ansprüche, worin das Atomverhältnis von Brom (errechnet als elementares Brom) in der Bromkomponente des Katalysators zu Kobalt und Mangan (errechnet als elementares Kobalt und elementares Mangan) in den Kobalt- und Mangan-Komponenten des Katalysators im Bereich von 0,075 bis 0,4 Milligramm-Atom pro Milligramm-Atom der Gesamtmenge an Kobalt und Mangan liegt.

**5.** Verfahren nach einem der vorhergehenden Ansprüche, worin das Atomverhältnis von Cer (errechnet als elementares Cer) in der Cer-Komponente des Katalysators zu Kobalt (errechnet als elementares Kobalt) in der Kobalt-Komponente des Katalysators im Bereich von 0,05 bis 0,6 Milligramm-Atom pro Milligramm-Atom Kobalt liegt.

**6.** Verfahren nach einem der vorhergehenden Ansprüche, worin der Sauerstoff in dem Reaktor in einer solchen Konzentration gehalten wird, daß der Sauerstoffgehalt in den abgeleiteten Dämpfen von 0,05 bis 8 Volumen-% beträgt.

**7.** Verfahren nach einem der vorhergehenden Ansprüche, worin die Oxidation bei einer Temperatur im Bereich von 165 °C bis 270 ° C erfolgt.

**8.** Verfahren nach Anspruch 7, worin die Oxidation bei einer Temperatur im Bereich von 170 °C bis 200 ° C erfolgt.

**9.** Verfahren nach einem der vorhergehenden Ansprüche, worin das Lösungsmittel eine Mischung aus Essigsäure und von 1 bis 30 Gew.-% Wasser, bezogen auf das Gewicht der Essigsäure, ist.

**10.** Verfahren nach Anspruch 9, worin das Lösungsmittel eine Mischung aus Essigsäure und von 2 bis 15 Gew.-% Wasser, bezogen auf das Gewicht der Essigsäure, ist.

**11.** Verfahren nach einem der vorhergehenden Ansprüche, worin der Druck im Bereich von 0 bis 35 $kg/cm^2$ Manometerdruck liegt.

**12.** Verfahren nach Anspruch 11, worin der Druck im Bereich von 10 bis 30 $kg/cm^2$ Manometerdruck liegt.

## Revendications

**1.** Procédé de préparation d'acide 2,6-naphtalène dicarboxylique consistant à soumettre à une oxydation exothermique du 2,6-diéthylnaphtalène ou son dérivé par oxydation comme matière de départ avec un gaz contenant de l'oxygène dans la phase liquide dans un solvant comprenant un acide monocarboxylique aliphatique, dans un réacteur d'oxydation à une température de 165 à 270 °C et une pression de 101 à 3534 kPa (0-35 $kg/cm^2$ manométriques) et en présence d'un catalyseur d'oxydation comprenant des composants de cobalt, de manganèse, de brome et de cérium, dans lequel le rapport atomique du cobalt (calculé en cobalt élémentaire) dans le composant de cobalt du catalyseur au matériau de départ dans l'oxydation en phase liquide se situe dans la plage de 30 à 10.000 mga/g-mole du matériau de départ, le rapport atomique du manganèse (calculé en manganèse élémentaire) dans le composant de manganèse du catalyseur au cobalt (calculé en cobalt élémentaire) dans le composant de cobalt du catalyseur se situe dans la plage de 0,5 à 3 mga par mga de cobalt, le rapport atomique du brome (calculé en brome élémentaire) dans le composant de brome du catalyseur à la teneur totale de cobalt et de manganèse (calculée en cobalt élémentaire et en manganèse élémentaire) dans les composants de cobalt et de manganèse du catalyseur se situe dans la plage de 0,05 à 1 mga par mga de la teneur totale en cobalt et en manganèse et le rapport atomique du cérium (calculé en cérium élémentaire) dans le composant de cérium du catalyseur au cobalt (calculé en cobalt élémentaire) dans le composant de cobalt du catalyseur se situe dans la plage de 0,025 à 1,0 mga par mga de cobalt, la chaleur générée dans le réacteur d'oxydation est au moins partiellement dissipée par vaporisation des liquides qui s'y trouvent et retrait des vapeurs obtenues du réacteur d'oxydation, et l'oxygène est maintenu à un niveau de concentration tel dans le réacteur d'oxydation que la concentration d'oxygène dans les vapeurs retirées précitées se situe dans la plage de 0,1 à 15 % en volume.

**2.** Procédé selon la revendication 1, dans lequel le rapport atomique du cobalt (calculé en cobalt élémentaire) dans le composant de cobalt du catalyseur au matériau de départ dans l'oxydation en phase liquide se situe dans la plage de 60 à 500 mga par g-mole du matériau de départ.

**3.** Procédé selon la revendication 1 ou 2, dans lequel le rapport atomique du manganèse (calculé en manganèse élémentaire) dans le composant de manganèse du catalyseur au cobalt (calculé en cobalt élémentaire) dans le composant de cobalt du catalyseur se situe dans la plage de 1,0 à 2,5 mga par mga de cobalt.

**4.** Procédé selon l'une quelconque des revendications précédentes, dans lequel le rapport atomique du brome (calculé en brome élémentaire) dans le composant de brome du catalyseur à la teneur totale en cobalt et manganèse (calculée en cobalt élémentaire et en manganèse élémentaire) dans les composants de cobalt et de manganèse du catalyseur se situe dans la plage de 0,075 à 0,4 mga par mga de la teneur totale en cobalt et en manganèse.

**5.** Procédé selon l'une quelconque des revendications précédentes, dans lequel le rapport atomique du cérium (calculé en cérium élémentaire) dans le composant de cérium du catalyseur au cobalt (calculé en cobalt élémentaire) dans le composant de cobalt du catalyseur se situe dans la plage de 0,05 à 0,6 mga par mga de cobalt.

**6.** Procédé selon l'une quelconque des revendications précédentes, dans lequel l'oxygène est maintenu à un niveau de concentration dans le réacteur tel que la concentration en oxygène des vapeurs retirées se situe dans la plage de 0,05 à 8 % en volume.

**7.** Procédé selon l'une quelconque des revendications précédentes, dans lequel l'oxydation est réalisée à une température dans la plage de 165 à 270 ° C.

**8.** Procédé selon la revendication 7, dans lequel l'oxydation est réalisée à une température dans la plage de 170 à 200 ° C.

**9.** Procédé selon l'une quelconque des revendications précédentes, dans lequel le solvant est un mélange d'acide acétique et de 1 à 30 % en poids d'eau sur base du poids de l'acide acétique.

**10.** Procédé selon la revendication 9, dans lequel le solvant est un mélange d'acide acétique et de 2 à 15 % en poids d'eau sur base du poids de l'acide acétique.

**11.** Procédé selon l'une quelconque des revendications précédentes, dans lequel la pression se situe dans la plage de 0 à 35 kg/cm$^2$ manométriques.

**12.** Procédé selon la revendication 11, dans lequel la pression se situe dans la plage de 10 à 30 kg/cm$^2$ manométriques.